# EUROPEAN PATENT SPECIFICATION

(11) **EP 3 244 330 B1**
(45) Date of publication and mention of the grant of the patent: **29.04.2020**
(21) Application number: 17170006.5
(22) Date of filing: 08.05.2017
(51) Int. Cl.: A61B 17/072, A61B 17/29

(54) **ADAPTER ASSEMBLY WITH PULLEY SYSTEM AND WORM GEAR DRIVE FOR INTERCONNECTING ELECTROMECHANICAL SURGICAL DEVICES AND SURGICAL END EFFECTORS**
ADAPTERANORDNUNG MIT RIEMENSCHEIBENSYSTEM UND SCHNECKENANTRIEB ZUR VERBINDUNG ELEKTROMECHANISCHER CHIRURGISCHER VORRICHTUNGEN UND CHIRURGISCHER ENDEFFEKTOREN
ENSEMBLE ADAPTATEUR COMPORTANT UN SYSTÈME DE POULIE ET ENTRAÎNEMENT À ENGRENAGE À VIS SANS FIN PERMETTANT D'INTERCONNECTER DES DISPOSITIFS CHIRURGICAUX ÉLECTROMÉCANIQUES ET DES EFFECTEURS D'EXTRÉMITÉ CHIRURGICAUX

(30) Priority: 09.05.2016 US 201662333584 P; 19.04.2017 US 201715491046
(43) Date of publication of application: 15.11.2017
(73) Proprietor: Covidien LP, Mansfield, MA 02048 (US)
(72) Inventor: NICHOLAS, David, Trumbull, CT 06611 (US)
(74) Representative: Maschio & Soames IP Ltd

(56) References cited:
- EP-A1- 3 195 812
- EP-A2- 1 980 214
- US-B2- 8 556 152

## Description

### CROSS-REFERENCE TO RELATED APPLICATIONS

This application claims the benefit of and priority to U.S. Provisional Patent Application Serial No. 62/333,584, filed May 9, 2016.

### TECHNICAL FIELD

The present disclosure relates to adapter assemblies for use in surgical systems. More specifically, the present disclosure relates to adapter assemblies for use with, and to electrically and mechanically interconnect, electromechanical surgical devices and surgical end effectors, and to surgical systems including handheld electromechanical surgical devices and adapter assemblies for connecting surgical end effectors to the handheld electromechanical surgical devices.

### BACKGROUND

A number of surgical device manufacturers have developed product lines with proprietary powered drive systems for operating and/or manipulating a surgical device. In many instances the surgical devices include a powered handle assembly, which is reusable, and a disposable end effector or the like that is selectively connected to the powered handle assembly prior to use and then disconnected from the end effector following use in order to be disposed of or in some instances, sterilized for re-use.

Many of the existing end effectors for use with many of the existing powered surgical devices and/or handle assemblies are driven by a linear force. For example, end effectors for performing endo-gastrointestinal anastomosis procedures, end-to-end anastomosis procedures and transverse anastomosis procedures, each typically require a linear driving force in order to be operated. These end effectors are not compatible with surgical devices and/or handle assemblies that use a rotary motion to deliver power or the like.

In order to make the linear driven end effectors compatible with powered surgical devices and/or handle assemblies that use a rotary motion to deliver power, adapters and/or adapter assemblies are used to interface between and interconnect the linear driven end effectors with the powered rotary driven surgical devices and/or handle assemblies. Many of these adapter and/or adapter assemblies are complex devices including many parts and requiring extensive labor to assemble.

Adapter concepts often include a cable system for distal rotation and/or articulation. Some designs of cable systems include pulleys or lead screws with counter-directional threads to generate linear motion. Consistent with stroke dynamics, lead screw designs can require additional length to accommodate coordinated cable take-up and release. By comparison, pulley designs can be more compact than lead screw designs, but typically require different considerations including those associated with assembly and tensioning.

Accordingly, a need exists to develop adapters and/or adapter assemblies that incorporate fewer parts, are less labor intensive to assemble, and are ultimately more economical to manufacture. Specifically, a need exits to develop such adapters and/or adapter assemblies with improved pulley designs that simplify manufacturing and assembly as well as improve cable tensioning. US8556152B2 discloses an adapter assembly for selectively interconnecting an end effector that is configured to perform a function. EP3195812A1 is a document according to Article 54(3) EPC.

### SUMMARY

According to an aspect of the present disclosure, an adapter assembly is provided. The adapter assembly selectively interconnects an end effector that is configured to perform a function and a surgical device that is configured to operate the end effector. The adapter assembly includes an outer tube having a distal end and a proximal end, a housing secured to the proximal end of the outer tube, and a cable drive assembly supported by the housing.

The cable drive assembly includes a worm gear, a cable gear coupled to the worm gear and rotatable in response to rotation of the worm gear, a capstan coupled to the cable gear and rotatable in response to rotation of the cable gear, and a cable coupled to the capstan. The cable may be axially translatable in response rotation of the capstan to actuate a function of the end effector while connected to the distal end of the outer tube.

In certain embodiments of the adapter assembly, the cable drive assembly may include a second worm gear, a second cable gear coupled to the second worm gear, a second capstan coupled to the second cable gear, and a second cable coupled to the second capstan. The second cable may be axially translatable in response to rotation of one or more of the second worm gear, the second cable gear, and the second capstan.

In some embodiments of the adapter assembly, the cable drive assembly may further include one or more pulleys supporting the cable and configured to direct the cable into the outer tube.

In certain embodiments of the adapter assembly, the cable drive assembly may further include a body portion that supports the worm gear and the cable gear in contacting relation with one another.

The adapter assembly may further include a firing assembly that extends through the cable drive assembly and into the outer tube. In some embodiments, the firing assembly may include a firing shaft that rotates independent of the cable drive assembly to actuate a firing function of the end effector.

The housing of the adapter assembly may include an outer housing and an inner housing that support the cable drive assembly therein.

In some embodiments of the adapter assembly, the outer tube defines a longitudinal axis that extends between the proximal and distal ends of the outer tube. The worm gear may be supported on a shaft member that extends in parallel relationship to the longitudinal axis of the outer tube. The shaft member may be rotatable to rotate the worm gear.

According to another aspect of the present disclosure, a surgical stapling apparatus is provided. The surgical stapling apparatus includes an end effector, a surgical device configured to operate the end effector, and an adapter assembly for selectively interconnecting the end effector and the surgical device.

The adapter assembly of the surgical stapling apparatus includes an outer tube having a distal end and a proximal end, a housing secured to the proximal end of the outer tube, and a cable drive assembly supported by the housing. The cable drive assembly includes a worm gear, a cable gear coupled to the worm gear, a capstan coupled to the cable gear, and a cable coupled to the capstan. The cable may be axially translatable in response rotation of one or more of the worm gear, the cable gear, and the capstan.

The adapter assembly of the surgical stapling apparatus may further include a firing assembly that extends through the cable drive assembly and into the outer tube. The firing assembly may include a firing shaft that rotates independent of the cable drive assembly to actuate a firing function of the end effector.

In some embodiments of the surgical stapling apparatus, the cable drive assembly may further include a second worm gear, a second cable gear coupled to the second worm gear, a second capstan coupled to the second cable gear, and a second cable coupled to the second capstan. The second cable may be axially translatable in response to rotation of one or more of the second worm gear, the second cable gear, and the second capstan.

In certain embodiments of the surgical stapling apparatus, the housing of the adapter assembly may include an outer housing and an inner housing. The inner and outer housings may support the cable drive assembly therein.

In some embodiments of the surgical stapling apparatus, the cable drive assembly may further include one or more pulleys supporting the cable and configured to direct the cable into the outer tube.

In certain embodiments of the surgical stapling apparatus, the cable drive assembly may further include a body portion that supports the worm gear and the cable gear in contacting relation with one another.

In some embodiments of the surgical stapling apparatus, the outer tube of the adapter assembly defines a longitudinal axis that extends between the proximal and distal ends of the outer tube. The worm gear may be supported on a shaft member that extends in parallel relationship to the longitudinal axis of the outer tube. The shaft member may be rotatable to rotate the worm gear.

According to yet another aspect of the present disclosure, an adapter assembly for selective connection to a surgical device is provided. The adapter assembly includes an outer tube having a distal end and a proximal end, a housing secured to the proximal end of the outer tube, and a cable drive assembly supported by the housing. The cable drive assembly includes a worm gear drive assembly, a cable gear assembly coupled to the worm gear drive assembly, and one or more cables coupled to the cable gear assembly and axially translatable within the outer tube.

In some embodiments, the cable drive assembly may further include a second worm gear, a second cable gear coupled to the second worm gear, a second capstan coupled to the second cable gear, and a second cable coupled to the second capstan.

Further details and aspects of exemplary embodiments of the present disclosure are described in more detail below with reference to the appended figures.

### BRIEF DESCRIPTION OF THE DRAWINGS

The accompanying drawings, which are incorporated in and constitute a part of this specification, illustrate embodiments of the disclosure and, together with a general description of the disclosure given above, and the detailed description of the embodiment(s) given below, serve to explain the principles of the disclosure, wherein:
FIG. 1 is a perspective view of an electromechanical surgical system in accordance with the principles of the present disclosure;
FIG. 2A is a perspective view of an adapter assembly of the electromechanical surgical system of FIG. 1;
FIG. 2B is a perspective view of the indicated area of detail shown in FIG. 2A;
FIG. 3 is an enlarged, top, perspective view of a proximal portion of the adapter assembly of FIG. 2, the proximal portion of the adapter assembly shown with a portion of an outer housing thereof removed for clarity;
FIG. 4 is a perspective view, with parts separated, of the proximal portion of the adapter assembly of FIG. 2;
FIG. 5 is an enlarged, perspective view, of the indicated area of detail shown in FIG. 4;
FIG. 6 is an enlarged, perspective view of a distal housing of the adapter assembly of FIG. 2;
FIG. 7 is a perspective view, with parts separated, of a drive system of the adapter assembly of FIG. 2;
FIG. 8 is a cross-sectional view of the adapter assembly of FIG. 2 as taken along section line 8-8 shown in FIG. 2;
FIG. 9 is a cross-sectional view of a portion of the adapter assembly of FIG. 2 as taken along section line 9-9 shown in FIG. 8;
FIG. 10 is a cross-sectional view of a portion of the adapter assembly of FIG. 2 as taken along section line 10-10 shown in FIG. 8;
FIG. 11 is a perspective view, with parts separated, of an end effector of the electromechanical surgical system of FIG. 1;
FIG. 12 is a perspective view illustrating cables of the drive system of FIG. 7 being tensioned with tensioning devices;
FIG. 13 is a cross-sectional view of FIG. 11 as taken along section line 13-13 shown in FIG. 12; and
FIG. 14 is a perspective view of the drive system of FIG. 7 and of a tensioning device.

### DETAILED DESCRIPTION

Electromechanical surgical systems of the present disclosure include surgical devices in the form of powered handheld electromechanical instruments configured for selective attachment to a plurality of different end effectors that are each configured for actuation and manipulation by the powered handheld electromechanical surgical instrument. In particular, the presently described electromechanical surgical systems include adapter assemblies that interconnect the powered handheld electromechanical surgical instruments to the plurality of different end effectors. Each adapter assembly includes an articulation assembly and a firing assembly that is operatively coupled to a powered handheld electromechanical surgical instrument for effectuating actuation and/or manipulation of the plurality of different end effectors.

Embodiments of the presently disclosed electromechanical surgical systems, surgical devices/handle assemblies, adapter assemblies, and/or end effectors/loading units are described in detail with reference to the drawings, in which like reference numerals designate identical or corresponding elements in each of the several views. As used herein the term "distal" refers to that portion of the system, assembly, device, and/or component thereof, farther from the user, while the term "proximal" refers to that portion of the system, assembly, device, and/or component thereof, closer to the user.

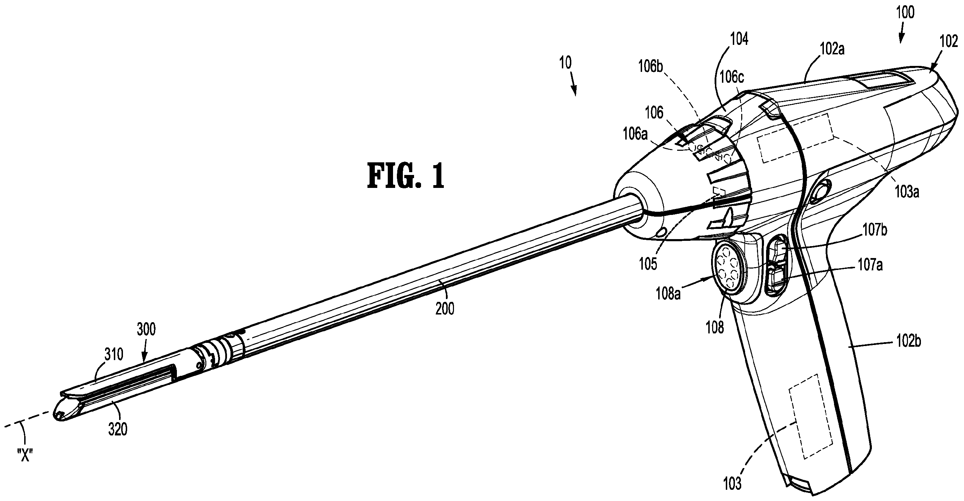

Turning now to FIG. 1, an electromechanical surgical system, in accordance with the present disclosure, generally referred to as 10, includes a surgical device 100 in the form of a powered handheld electromechanical instrument, an adapter assembly 200, and a surgical loading unit (e.g., multiple- or single-use loading unit) or end effector 300. Surgical device 100 is configured for selective connection with adapter assembly 200, and, in turn, adapter assembly 200 is configured for selective connection with end effector 300. Together, surgical device 100 and adapter assembly 200 may cooperate to actuate end effector 300.

Surgical device 100 of electromechanical surgical system 10 includes a handle housing 102 including a controller or circuit board (not shown) and a drive mechanism 106 situated therein. The circuit board is configured to control the various operations of surgical device 100. Handle housing 102 defines a cavity therein (not shown) for selective removable receipt of a rechargeable battery 103 therein. The battery 103 is configured to supply power to any electrical components of surgical device 100. The drive mechanism 106 within the handle housing 102 is configured to drive rotatable shafts 106a-106c (and/or gear components - not shown) within handle housing 102 in order to perform the various operations of surgical device 100. In particular, drive mechanism 106 (and/or components thereof) is operable to selectively articulate end effector 300 about a longitudinal axis "X" and relative to a distal end of adapter assembly 200, to selectively rotate end effector 300 about longitudinal axis "X" and relative to handle housing 102, to selectively move/approximate/separate an anvil assembly 310 and a cartridge assembly 320 of end effector 300 relative to one another, and/or to fire a stapling and cutting cartridge within cartridge assembly 320 of end effector 300.

Handle housing 102 of surgical device 100 includes an upper housing portion 102a that houses various components of surgical device 100, and a lower hand grip portion 102b extending from upper housing portion 102a. Lower hand grip portion 102b of handle housing 102 may be disposed distally of a proximal-most end of upper housing portion 102a of handle housing 102. The location of lower hand grip portion 102b relative to upper housing portion 102a is selected to balance a weight of surgical device 100 while surgical device 100 is connected to or supports adapter assembly 200 and/or end effector 300.

A connection portion 104 of handle housing 102 is configured to secure to a proximal end of adapter assembly 200. Connection portion 104 houses an articulation contact surface 105 in electrical communication with the circuit board (not shown) of surgical device 100 to control drive mechanism 106. Each rotatable drive shaft 106a-106c of drive mechanism 106 can be independently, and/or dependently, actuatable and rotatable. In embodiments, rotatable drive shafts, 106a, 106b, and 106c may be arranged in a common plane or line with one another. As can be appreciated, any number of rotatable drive shafts can be arranged in any suitable configuration.

Handle housing 102 of surgical device 100 supports finger-actuated control buttons, rocker devices, and/or the like for activating various functions of surgical device 100. For example, handle housing 102 may support actuators including an actuation pad 108 in operative registration with sensors 108a that cooperate with actuation pad 108 to effectuate, for instance, opening, closing, and/or firing of end effector 300. Handle housing 102 can support actuators 107a, 107b which can be disposed in electrical communication with one or more motors (not shown) of drive mechanism 106 to effectuate rotation of rotatable drive shafts 106a, 106b, and/or 106c for actuation thereof to enable adjustment of one or more of the components of adapter assembly 200. Any of the presently described actuators can have any suitable configuration (e.g., button, knob, toggle, slide, etc.).

Reference may be made to International Application No. PCT/US2008/077249, filed September 22, 2008 (Inter. Pub. No. WO 2009/039506), and U.S. Patent Application Publication No. 2011/0121049, filed on November 20, 2009, for a detailed description of various internal components of and operation of exemplary electromechanical surgical systems, the components of which are combinable and/or interchangeable with one or more components of electromechanical surgical systems 10 described herein.

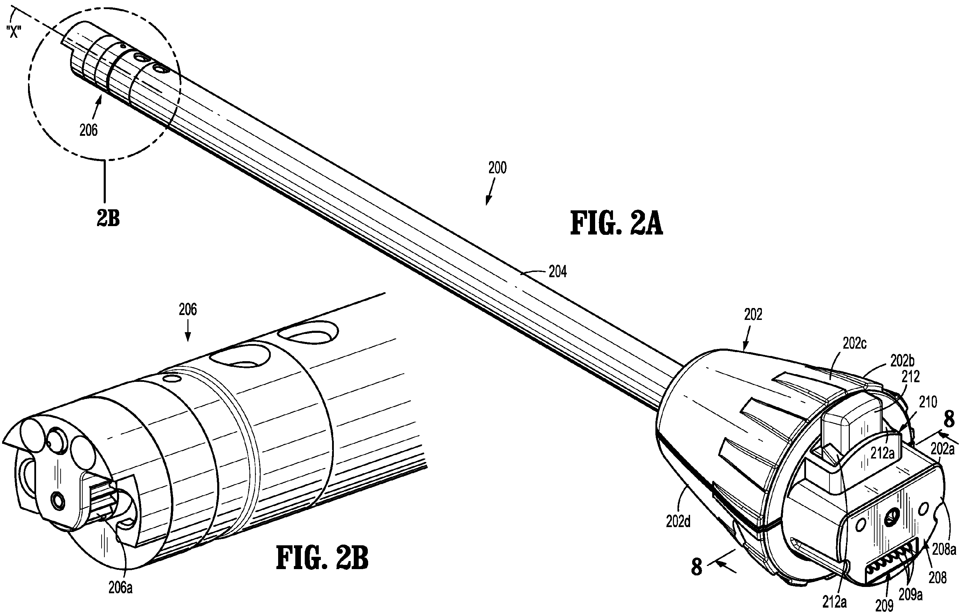

With reference to FIGS. 2A and 2B, adapter assembly 200 of electromechanical surgical system 10 includes a housing 202 at a proximal end portion thereof and an outer tube 204 that extends distally from housing 202 along longitudinal axis "X" to a distal end portion 206. Distal end portion 206 of outer tube 204 couples a distal end of adapter assembly 200 to a proximal end of end effector 300. Reference can be made to U.S. Patent Application Publication No. 2015/0297199, filed April 21, 2014 for a detailed description of exemplary distal end portions. As described in U.S. Patent Application Publication No. 2015/0297199, the distal end portion may support a gimbal or the like that couple to an articulation assembly such as the articulation or cable drive assembly described herein to enable end effectors, such as end effector 300 of electromechanical surgical system 10, to articulate relative to adapter assembly 200 of electromechanical surgical system 10. Such distal end portions 206 may support a rotatable gear 206a that engages with a proximal end of end effector 300 to effectuate a firing thereof as described in greater detail below.

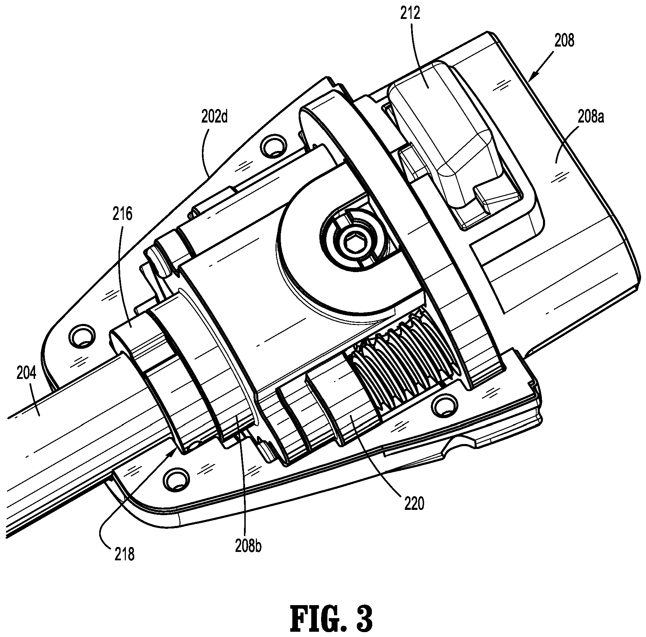

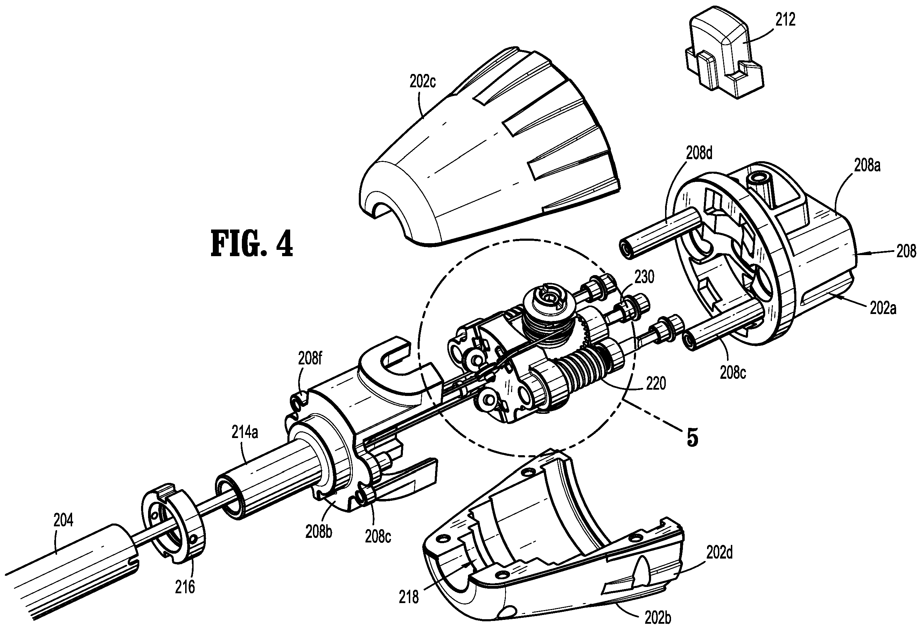

Referring to FIGS. 2A-4, housing 202 of adapter assembly 200 includes an inner housing 202a and an outer housing 202b having first and second housing halves 202c, 202d. Inner housing 202a includes a housing body 208 having a proximal housing body 208a and a distal housing body 208b that couple together via fastener-receiving arms 208c, 208d of proximal housing body 208a and fastener-receiving ears 208e, 208f of distal housing body 208b. Proximal housing body 208a of inner housing 202a supports an electrical assembly 209 therein and a mounting assembly 210 thereon.

Electrical assembly 209 of housing 202 may include a circuit board with contact pins 209a for electrical connection to a corresponding electrical plug (not shown) disposed in connection portion 104 of surgical device 100 (e.g., for calibration and communication of life-cycle information to the circuit board of the surgical device 100).

Mounting assembly 210 of housing 202 includes a mounting button 212 that is biased in an extended position and is configured to be depressed downwardly to a compressed position. In the compressed position, mounting button 212 is disposed in close approximation with housing body 208 of inner housing 202a and offset from the extended position thereof. Mounting button 212 includes sloped engagement features 212a that are configured to contact connection portion 104 (FIG. 1) of handle housing 102 while mounting button 212 is in the extended position to facilitate securement of housing 202 of adapter assembly 200 to connection portion 104 of handle housing 102. For a detailed description of similar electrical and mounting assemblies, reference can be made to U.S. Patent Application Publication No. 2015/0157320, filed November 21, 2014.

Outer housing 202b of housing 202 is disposed around inner housing 202a of housing 202 to support an articulation or cable drive assembly 220 and a firing assembly 230 within housing 202 of adapter assembly 200. Distal housing body 208b of inner housing 208 includes a distal shaft 214a that is received within a proximal end of outer tube 204 and coupled thereto by a bearing 216 mounted within a channel 218 defined within outer housing 202b of housing 202.

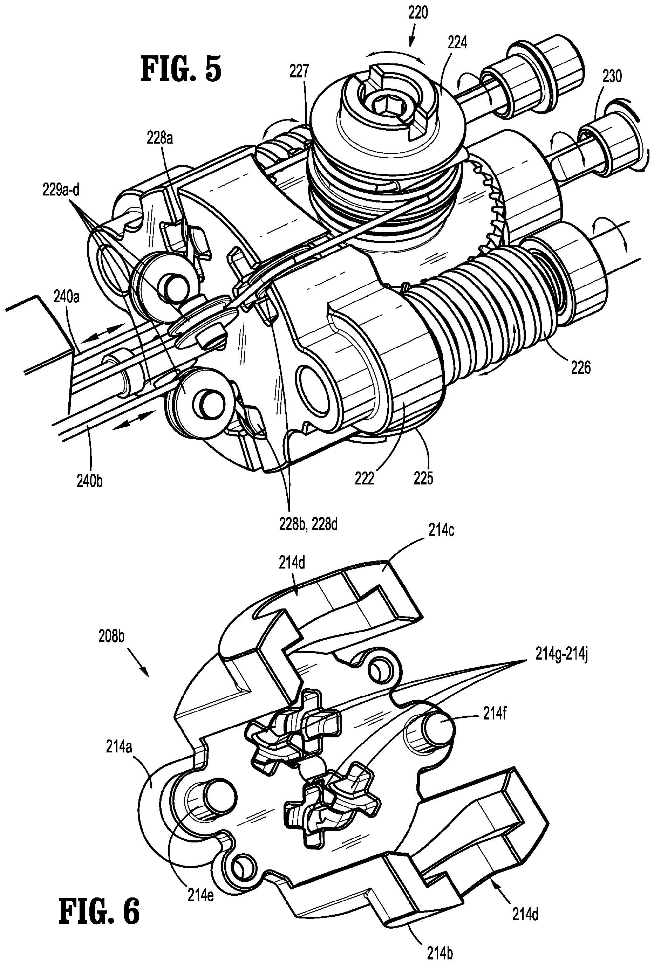

As seen in FIG. 6, distal housing body 208b further includes mirrored arms 214b, 214c each defining a U-shaped passage 214d. U-shaped passages 214d of distal housing body 208b extend through arms 214b, 214c of distal housing body 208b and are configured to receive first and second cable gear assemblies 224, 225 of cable drive assembly 220 therein. Distal housing body 208b also includes pins or bosses 214e, 214f that extend proximally from a proximal surface of distal housing body 208b. The proximal surface of distal housing body 208b also defines distal pulley recesses 214g-214j therein.

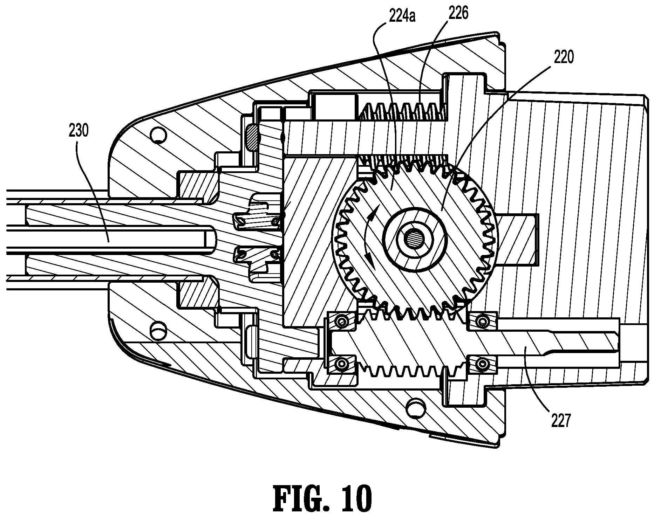

With reference to FIGS. 5-7 and 10, cable drive assembly 220 includes a body portion 222, a first cable gear assembly 224, a second cable gear assembly 225, a first worm gear drive assembly 226, and a second worm gear drive assembly 227, proximal guide pulleys 228a-228d, and distal guide pulleys 229a-229d.

Body portion 222 of cable drive assembly 220 defines proximal pulley recesses 222a-222d that receive respective proximal guide pulleys 228a-228d therein to enable the respective proximal guide pulleys 228a-228d to rotate therein as cables 240a, 240b of cable drive assembly 220 rotate around respective proximal guide pulleys 228a-228d to manipulate end effector 300. Similarly, distal guide pulleys 229a-229d of cable drive assembly 220 are received within respective distal pulley recesses 214g-214j of distal housing body 208b of inner housing 202a to enable distal guide pulleys 229a-229d to rotate therein as cables 240a, 240b of cable drive assembly 220 rotate around respective distal guide pulleys 229a-229d to manipulate end effector 300.

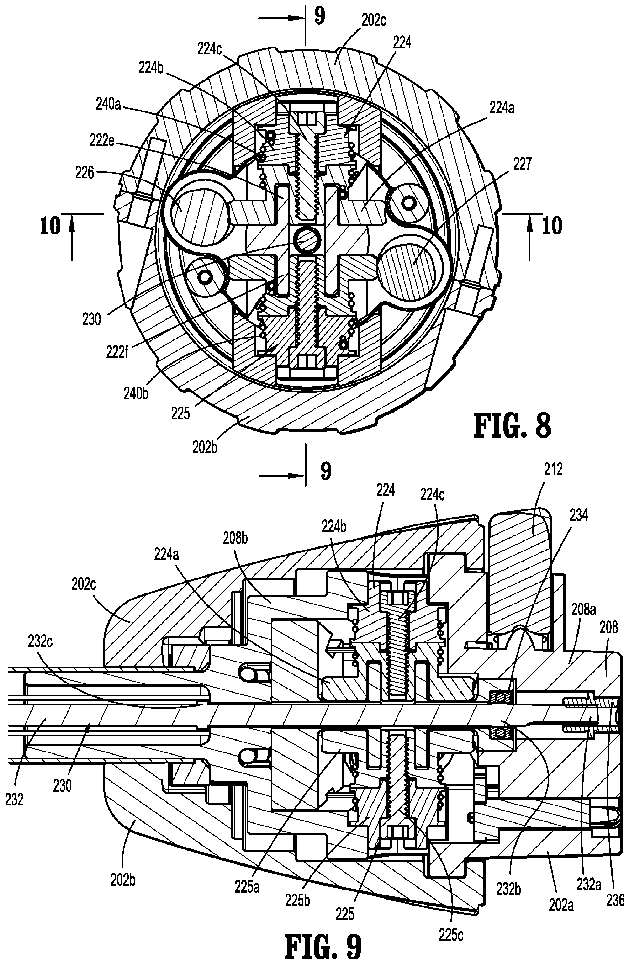

Body portion 222 of cable drive assembly 220 includes an upper mounting projection 222e extending therefrom and positioned to partially receive first cable gear assembly 224 of cable drive assembly 220 therein for supporting first cable gear assembly 224 on upper mounting projection 222e of body portion 222. A lower mounting projection 222f (see FIG. 8) also extends from body portion 222 of cable drive assembly 220 in a direction opposite upper mounting projection 222e of body portion 222. Lower mounting projection 222f of body portion 222 is positioned to support second cable gear assembly 225 of cable drive assembly 220 thereon. Body portion 222 of cable drive assembly 220 further defines worm gear recesses 222g, 222h therein that rotatably receive first and second worm drive assemblies 226, 227, respectively in a proximal end thereof and pins 214e, 214f of distal housing body 208b in a distal end thereof. A firing shaft passage 222i is defined centrally through body portion 222 to receive a firing assembly 230 therein.

Referring to FIGS. 5, 7 and 8, first cable gear assembly 224 of cable drive assembly 220 includes an upper gear 224a, an upper capstan 224b supported on upper gear 224a, and an upper fastener 224c that couples upper capstan 224b to upper gear 224a while upper capstan 224b is coupled to upper mounting projection 222e of body portion 222 of cable drive assembly 220. Similarly, second cable gear assembly 225, which mirrors first cable gear assembly 224, includes a lower gear 225a, a lower capstan 225b supported on lower gear 225a, and a lower fastener 225c that couples lower capstan 225b to lower gear 225a while lower capstan 225b is coupled to lower mounting projection 222f of body portion 222 of cable drive assembly 220. Each of upper and lower gears 224a, 225a of respective first and second gear assemblies 224, 225 include a center protuberance 2245 that is received in respective upper and lower mounting projections 222e, 222f of body portion 222 to enable respective first and second gear assemblies 224, 225 to rotate about respective upper and lower mounting projections 222e, 222f of body portion 222. First and second cables 240a, 240b are wound around respective upper and lower capstans 224b, 225b and around respective proximal and distal guide pulleys 228a-228d, 229a-229d so that opposite ends/sides of each of the respective cables 240a, 240b extends distally through outer tube 204 to operatively couple to end effector 300 (e.g., to effectuate rotation and/or articulation thereof).

First worm gear drive assembly 226 of cable drive assembly 220 includes a first worm drive 226a rotatably supported between bearings 226b, 226c. First worm drive 226a includes a worm gear 226d secured on a shaft member 226e. Shaft member 226e has a proximal driving end 226f received in bearing 226b and a distal end 226g received in bearing 226c.

Similarly, second worm gear drive assembly 227 of cable drive assembly 220 includes a first worm drive 227a rotatably supported between bearings 227b, 227c. Second worm drive 227a includes a worm gear 227d secured on a shaft member 227e. Shaft member 227e has a proximal driving end 227f received in bearing 227b and a distal end 227g received in bearing 227c.

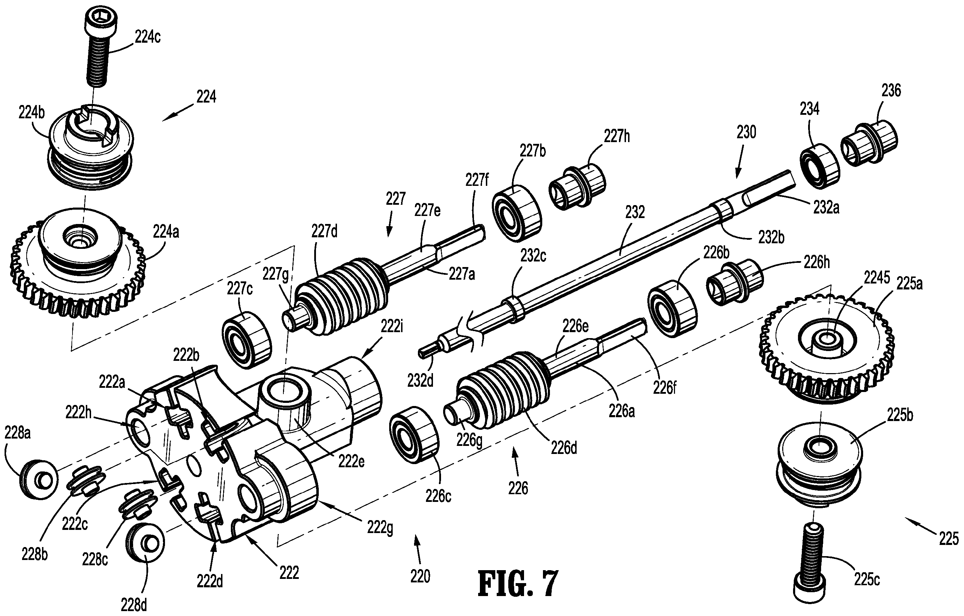

Referring to FIGS. 7, 9, and 10, firing assembly 230 of adapter assembly 200 includes a firing shaft 232, a bearing 234 supported on firing shaft 232, and an input socket 236 secured to a proximal end 232a of firing shaft 232. Firing shaft 232 of firing assembly 230 includes spaced collars 232b, 232c and a distal driving end 232d. Collar 232b of firing shaft 232 supports bearing 234 thereon and collar 232c of firing shaft 232 supports firing shaft 232 against distal housing body 208b of inner housing 202a. Distal driving end 232d of firing shaft 232 is extends to distal end portion 206 of outer tube 204 to effectuate a firing of end effector 300 as described in greater detail below.

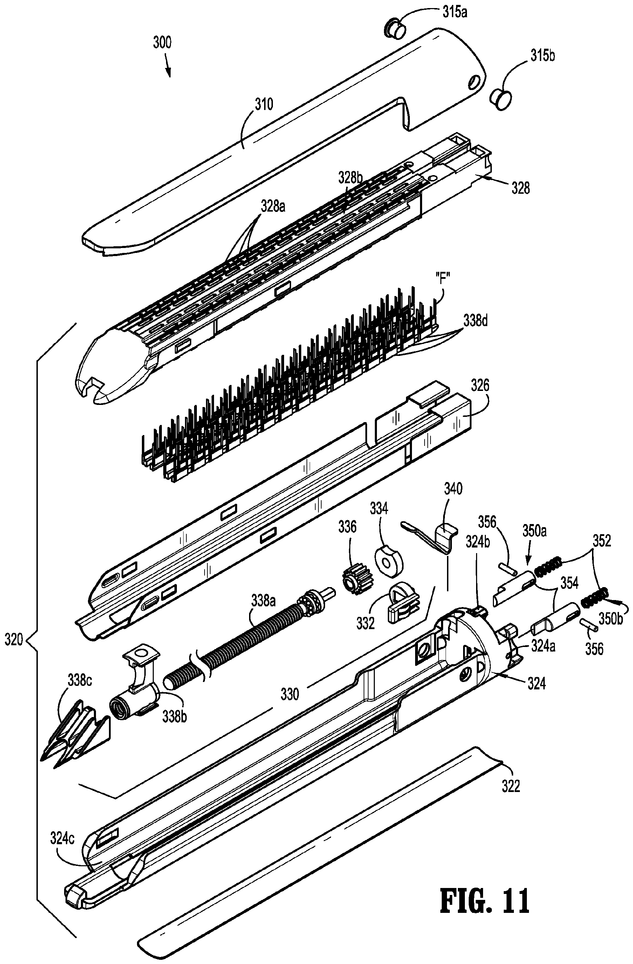

Turning now to FIG. 11, an embodiment of an end effector 300 is shown. End effector 300 includes an anvil 310 and a cartridge assembly 320 that are pinned together by pins 315a, 315b and movable between open and closed conditions. Anvil 310 and cartridge assembly 320 cooperate to apply linear rows of fasteners "F" (e.g., staples). In certain embodiments, fasteners "F" are of various sizes, and, in certain embodiments, fasteners "F" are loaded into various lengths or rows of cartridge assembly 320 of end effector 300 (e.g., about 30, 45 and 60 mm in length).

Cartridge assembly 320 of end effector 300 includes a base 322 secured to a mounting portion 324, a frame portion 326, and a cartridge portion 328. Cartridge portion 328 has a tissue engaging surface that defines fastener retaining slots 328a and a knife slot 328b therein. Mounting portion 324 of cartridge assembly 320 has mating surfaces 324a, 324b on a proximal end thereof and defines a receiving channel 324c therein that supports frame portion 326, cartridge portion 328, and a fastener firing assembly 330 therein. Cartridge assembly 320 supports a biasing member 340 (e.g., a leaf spring) that engages anvil 310.

Fastener firing assembly 330 of end effector 300 includes an electrical contact member 332 for electrical communication with the circuit board of surgical device 100, a bearing member 334, a gear member 336 that engages rotatable gear 206a of adapter assembly 200, and a screw assembly 338. Screw assembly 338 of fastener firing assembly 330 includes a lead screw 338a, a drive beam 338b, and an actuation sled 338c that is engagable with pusher members 338d.

Cartridge assembly 320 of end effector 300 also supports plunger assemblies 350a, 350b. Each of plunger assemblies 350a, 350b includes a spring 352, a plunger 354, and a pin 356 that secures each plunger assembly to mounting portion 324 of cartridge assembly 320. Plunger assemblies 350a, 350b cooperate with the proximal end of cartridge portion 328 to facilitate securement of cartridge portion 328 within mounting portion 324.

In order to secure the proximal end of end effector 300 to distal end portion 206 of outer tube 204 of adapter assembly 200, the proximal end of end effector 300 is aligned with distal end portion 206 of adapter assembly 200 so that the proximal end of end effector 300 can be coupled to distal end portion 206 of adapter assembly 200 such that mating surfaces 324a and 324b of end effector 300 engage with distal end portion 206 of adapter assembly 200 and the teeth of gear member 336 of end effector 300 enmesh with the teeth of rotatable gear 206a of distal end portion 206 of adapter assembly 200.

In use, actuation pad 108 of surgical device 100 is actuated to rotate one or both of rotatable drive shafts 106a, 106c (e.g., clockwise and/or counterclockwise) of surgical device 100 via motors (not shown) disposed within surgical device 100.

Rotation of rotatable drive shaft 106a of surgical device 100 causes a corresponding rotation of worm gear 227d of worm drive assembly 227 and thus, rotation of lower gear 225a of gear assembly 225. Rotation of lower gear 225a of gear assembly 225 rotates lower capstan 225b of gear assembly 225 to draw/retract/tighten one side/end of cable 240a of cable drive assembly 220 while letting out/releasing the opposite side/end of cable 240a. Similarly, rotation of rotatable drive shaft 106c of surgical device 100 causes a corresponding rotation of worm gear 226d of worm drive assembly 226 and thus, rotation of upper gear 224a of gear assembly 226. Rotation of upper gear 224a of gear assembly 224 rotates upper capstan 224b of gear assembly 224 to draw/retract/tighten one side/end of cable 240b of cable drive assembly 220 while letting out/releasing the opposite side/end of cable 240b. Cables 240a, 240b of cable drive assembly 200 can be drawn/retracted/tightened and/or let out/released as desired to effectuate articulation (e.g., a pitch and/or a yaw) of end effector 300 about longitudinal axis "X" of adapter assembly 200.

To fire fasteners "F" from end effector 300, actuation pad 108 of surgical device 100 is actuated to rotate rotatable drive shaft 106b via a motor 103a (see FIG. 1) within handle housing 102, and to effectuate rotation of firing shaft 232 of firing assembly 230 about longitudinal axis "X" of adapter assembly 200. Rotation of firing shaft 232 of firing assembly 230 rotates rotatable gear 206a of distal end portion 206 of adapter assembly 200, which in turn, causes rotation of gear member 336 of end effector 300.

Rotation of gear member 336 of firing assembly 330 rotates lead screw 338a of firing assembly 330 and enables drive beam 338b of firing assembly 330 to axially advance along lead screw 338a and through longitudinal knife slot 328b of cartridge portion 328 by virtue of a threaded engagement between lead screw 338a and drive beam 338b. Drive beam 338b of firing assembly 330 engages anvil 310 of end effector 300 to maintain anvil 310 and cartridge assembly 320 of end effector 300 in approximation. Distal advancement of drive beam 338b of firing assembly 330 advances actuation sled 338c of firing assembly 330 into engagement with pusher members 338d of end effector 300 and fires the fasteners "F" from fastener retention slots 328a of cartridge portion 328 for forming against corresponding fastener forming pockets (not shown) defined within anvil 310. End effector 300 can be reset and cartridge portion 328 of cartridge assembly 320 can be replaced so that end effector 300 can then be re-fired as needed or desired.

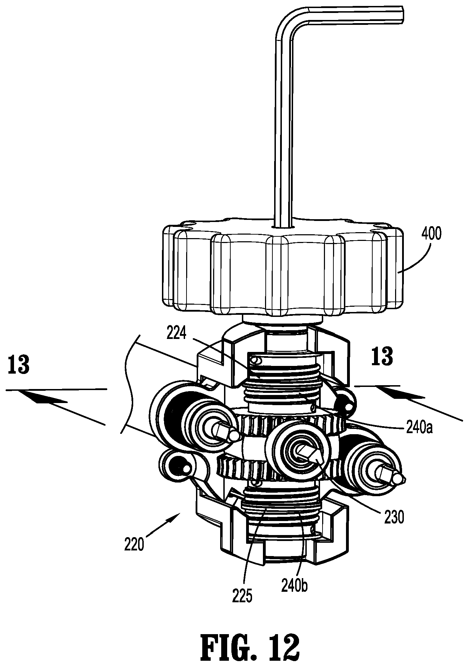

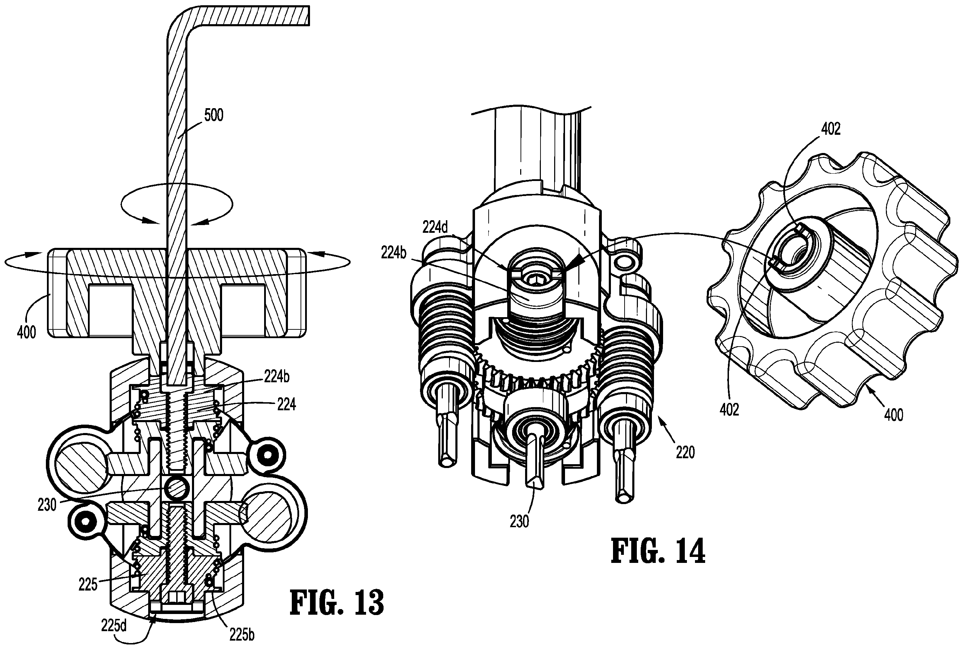

Turning now to FIGS. 12-14, upper capstan 224b of first cable gear assembly 224 defines a slot 224d therein and lower capstan 225b of second cable gear assembly 225 defines a slot 225d therein. Slots 224d, 225d of respective upper and lower capstans 224b, 225b are configured to selectively receive detents 402 of a rotatable knob 400 therein to enable tension in cables 240a, 240b to be adjusted upon rotation of upper and/or lower capstans 224b, 225b via rotation of rotatable knob 400. Rotatable knob 400 further defines a central channel 404 therethrough configured to selectively receive a fastener driver 500 (e.g., an Allen wrench) therethrough for tightening and/or loosening respective upper and lower fasteners 224c, 225c of respective first and second gear assemblies 224, 225 to further facilitate tension adjustments as needed or desired.

Persons skilled in the art will understand that the structures and methods specifically described herein and shown in the accompanying figures are non-limiting exemplary embodiments, and that the description, disclosure, and figures should be construed merely as exemplary of particular embodiments. It is to be understood, therefore, that the present disclosure is not limited to the precise embodiments described, and that various other changes and modifications may be effected by one skilled in the art without departing from the scope of the disclosure. Additionally, the elements and features shown or described in connection with certain embodiments may be combined with the elements and features of certain other embodiments without departing from the scope of the present disclosure, and that such modifications and variations are also included within the scope of the present disclosure. Accordingly, the subject matter of the present disclosure is not limited by what has been particularly shown and described.

## Claims

1. An adapter assembly (200) for selectively interconnecting an end effector (300) that is configured to perform a function and a surgical device (100) that is configured to operate the end effector (300), the adapter assembly (200) comprising:
an outer tube (204) having a distal end (206) and a proximal end;
a housing (202) secured to the proximal end of the outer tube (204); and
a cable drive assembly (220) supported by the housing (202) and including:
a worm gear (227d);
a cable gear (224a) coupled to the worm gear (227d) and rotatable in response to rotation of the worm gear (227d);
a capstan (224b) coupled to the cable gear (224a) and rotatable in response to rotation of the cable gear (224a); and
a cable (240a) coupled to the capstan (224b), the cable (240a) axially translatable in response rotation of the capstan (224b) to actuate a function of the end effector (300) while connected to the distal end (206) of the outer tube (204),
**characterised in that** the cable drive assembly (220) further includes a second worm gear (226d), a second cable gear (225a) coupled to the second worm gear (226d), a second capstan (225b) coupled to the second cable gear (225a), and a second cable (240b) coupled to the second capstan (225b), wherein the second cable (240b) is axially translatable in response to rotation of at least one of the second worm gear (226d), the second cable gear (225a), and the second capstan (225b),
wherein the capstan (224b) of the first cable gear (224a) defines a slot (224d) therein and the capstan (225b) of the second cable gear (225a) defines a slot (225d) therein, said slots (224d, 225d) being configured to selectively receive detents (402) of a rotatable knob (400) therein to enable tension in the cables (240a, 240b) to be adjusted upon rotation of the capstans (224b, 225b) via rotation of rotatable knob (400).

2. An adapter assembly (200) according to claim 1 wherein the rotatable knob 400 further defines a central channel (404) therethrough, which is configured to selectively receive a fastener driver (500) therethrough for tightening and/or loosening respective upper and lower fasteners (224c, 225c) that couple to the capstans (224b, 224b) to further facilitate tension adjustments.

3. The adapter assembly (200) of claim 1 or claim 2, further including a firing assembly (230) that extends through the cable drive assembly (220) and into the outer tube (204).

4. The adapter assembly (200) of claim 3, wherein the firing assembly (230) includes a firing shaft (232) that rotates independent of the cable drive assembly (220) to actuate a firing function of the end effector (300).

5. The adapter assembly (200) of any preceding claim, wherein the housing (202) includes an outer housing (202b) and an inner housing (202a), the inner and outer housings (202a, 202b) supporting the cable drive assembly (220) therein.

6. The adapter assembly (200) of any preceding claim, wherein the cable drive assembly (220) further includes at least one pulley (228a-d, 229a-d) supporting the cables (240a, 240b) and configured to direct the cables (240a, 240b) into the outer tube (204).

7. The adapter assembly (200) of any preceding claim, wherein the cable drive assembly (220) further includes a body portion (222) that supports the worm gears (226d, 227d) and the cable gears (224a, 225a) in contacting relation with one another.

8. The adapter assembly (200) of any preceding claim, wherein the outer tube (204) defines a longitudinal axis that extends between the proximal and distal ends (206) of the outer tube, and wherein the worm gears (226d, 227d) are supported on shaft members (226e, 227e) that extend in parallel relationship to the longitudinal axis of the outer tube (204), the shaft members (226e, 227e) being rotatable to rotate the worm gears (226d, 227d).

9. A surgical stapling apparatus, comprising:
an end effector (300);
a surgical device configured to operate the end effector (300); and an adapter assembly (200) according to any preceding claim.

## Patentansprüche

1. Adapterbaugruppe (200) zum selektiven Verbinden eines Endeffektors (300), der dafür konfiguriert ist, eine Funktion auszuführen, und einer chirurgischen Vorrichtung (100), die dafür konfiguriert ist, den Endeffektor (300) zu bedienen, miteinander, die Adapterbaugruppe (200) umfassend:
ein Außenrohr (204) mit einem distalen Ende (206) und einem proximalen Ende;
ein Gehäuse (202), das am proximalen Ende des Außenrohrs (204) befestigt ist; und
eine Kabelantriebsbaugruppe (220), die von dem Gehäuse (202) getragen wird und Folgendes beinhaltet:
ein Schneckengetriebe (227d);
ein Kabelgetriebe (224a), das mit dem Schneckengetriebe (227d) verbunden und in Reaktion auf eine Drehung des Schneckengetriebes (227d) drehbar ist;
eine Spillwinde (224b), die mit dem Kabelgetriebe (224a) verbunden und in Reaktion auf eine Drehung des Kabelgetriebes (224a) drehbar ist; und
ein Kabel (240a), das mit der Spillwinde (224b) verbunden ist, wobei das Kabel (240a) in Reaktion auf eine Drehung der Spillwinde (224b) axial verschiebbar ist, um eine Funktion des Endeffektors (300) zu betätigen, während er mit dem distalen Ende (206) des Außenrohrs (2004) verbunden ist,
**dadurch gekennzeichnet, dass**
die Kabelantriebsbaugruppe (220) ferner ein zweites Schneckengetriebe (226d), ein zweites Kabelgetriebe (225a), das mit dem zweiten Schneckengetriebe (226d) verbunden ist, eine zweite Spillwinde (225b), die mit dem zweiten Kabelgetriebe (225a) verbunden ist, und ein zweites Kabel (240b) umfasst, das mit der zweiten Spillwinde (225b) verbunden ist, wobei das zweite Kabel (240b) in Reaktion auf eine Drehung zumindest eines des zweiten Schneckengetriebes (226d), des zweiten Kabelgetriebes (225a) und der zweiten Spillwinde (225b) axial verschiebbar ist, wobei die Spillwinde (224b) des ersten Kabelgetriebes (224a) einen Schlitz (224d) darin definiert und die Spillwinde (225b) des zweiten Kabelgetriebes (225a) einen Schlitz (225d) darin definiert, wobei die Schlitze (224d, 225d) so konfiguriert sind, dass sie Rasten (402) eines drehbaren Knopfs (400) darin selektiv aufnehmen, um zu ermöglichen, dass eine Spannung in den Kabeln (240a, 240b) bei Drehung der Spillwinden (224b, 225b) durch Drehung des drehbaren Knopfs (400) eingestellt wird.

2. Adapterbaugruppe (200) nach Anspruch 1, wobei der drehbare Knopf 400 ferner einen mittleren Kanal (404) durch diesen definiert, der dafür konfiguriert ist, einen Befestigungsdreher (500) durch diesen selektiv aufzunehmen, um jeweilige obere und untere Befestigungselemente (224c, 225c) festzuziehen und/oder zu lockeren, die sich mit den Spillwinden (224b, 224b) verbinden, für eine noch leichtere Anpassung der Spannung.

3. Adapterbaugruppe (200) nach Anspruch 1 oder 2, die ferner eine Abschussbaugruppe (230) beinhaltet, die sich durch die Kabelantriebsbaugruppe (220) und in das Außenrohr (204) erstreckt.

4. Adapterbaugruppe (200) nach Anspruch 3, wobei die Abschussbaugruppe (230) eine Abschusswelle (232) beinhaltet, die sich unabhängig von der Kabelantriebsbaugruppe (220) dreht, um eine Abschussfunktion des Endeffektors (300) zu betätigen.

5. Adapterbaugruppe (200) nach einem vorstehenden Anspruch, wobei das Gehäuse (202) ein Außengehäuse (202b) und ein Innengehäuse (202a) beinhaltet, wobei das Innen- und das Außengehäuse (202a, 202b) die Kabelantriebsbaugruppe (220) darin tragen.

6. Adapterbaugruppe (200) nach einem vorstehenden Anspruch, wobei die Kabelantriebsbaugruppe (220) ferner zumindest eine Riemenscheibe (228a-d, 229a-d) beinhaltet, die die Kabel (240a, 240b) trägt und dafür konfiguriert ist, dass sie die Kabel (240a, 240b) in das Außenrohr (204) lenkt.

7. Adapterbaugruppe (200) nach einem vorstehenden Anspruch, wobei die Kabelantriebsbaugruppe (220) ferner einen Körperabschnitt (222) beinhaltet, der die Schneckengetriebe (226d, 227d) und die Kabelgetriebe (224a, 225a) in Kontakt miteinander trägt.

8. Adapterbaugruppe (200) nach einem vorstehenden Anspruch, wobei das Außenrohr (204) eine Längsachse definiert, die sich zwischen dem proximalen und dem distalen Ende (206) des Außenrohrs erstreckt, und wobei die Schneckengetriebe (226d, 227d) auf Wellenelementen (226e, 227e) getragen werden, die sich parallel zur Längsachse des Außenrohrs (204) erstrecken, wobei die Wellenelemente (226e, 227e) drehbar sind, um die Schneckengetriebe (226d, 227d) zu drehen.

9. Chirurgisches Klammersystem, das umfasst:
einen Endeffektor (300);
eine chirurgische Vorrichtung, die dafür konfiguriert ist, den Endeffektor (300) zu bedienen; und eine Adapterbaugruppe (200) nach einem vorstehenden Anspruch.

## Revendications

1. Ensemble adaptateur (200) permettant d'interconnecter de manière sélective un effecteur d'extrémité (300) qui est conçu pour exécuter une fonction et un dispositif chirurgical (100) qui est conçu pour faire fonctionner l'effecteur d'extrémité (300), l'ensemble adaptateur (200) comprenant :
un tube extérieur (204) ayant une extrémité distale (206) et une extrémité proximale ;
un boîtier (202) fixé à l'extrémité proximale du tube extérieur (204) ; et
un ensemble d'entraînement à câble (220) supporté par le boîtier (202) et comprenant :
un engrenage à vis sans fin (227d) ;
un engrenage à câble (224a) accouplé à l'engrenage à vis sans fin (227d) et pouvant tourner en réaction à la rotation de l'engrenage à vis sans fin (227d) ;
un cabestan (224b) accouplé à l'engrenage à câble (224a) et pouvant tourner en réaction à la rotation de l'engrenage à câble (224a) ; et
un câble (240a) accouplé au cabestan (224b), le câble (240a) pouvant être translaté axialement en réaction à la rotation du cabestan (224b) pour actionner une fonction de l'effecteur d'extrémité (300) tout en étant connecté à l'extrémité distale (206) du tube extérieur (204), **caractérisé en ce que**
l'ensemble d'entraînement à câble (220) comprend en outre une seconde vis sans fin (226d), un second engrenage à câble (225a) accouplé au second engrenage à vis sans fin (226d), un second cabestan (225b) accouplé au second engrenage à câble (225a), et un second câble (240b) accouplé au second cabestan (225b), dans lequel le second câble (240b) peut être translaté axialement en réaction à la rotation du second engrenage à vis sans fin (226d) et/ou du second engrenage à câble (225a) et/ou du second cabestan (225b),
dans lequel le cabestan (224b) du premier engrenage à câble (224a) définit une fente (224d) dans celui-ci et le cabestan (225b) du second engrenage à câble (225a) définit une fente (225d) dans celui-ci, lesdites fentes (224d, 225d) étant conçues pour recevoir de manière sélective les crans (402) d'un bouton rotatif (400) à l'intérieur de celles-ci afin de permettre le réglage de la tension des câbles (240a, 240b) lors de la rotation des cabestans (224b, 225b) par l'intermédiaire de la rotation du bouton rotatif (400).

2. Ensemble adaptateur (200) selon la revendication 1, dans lequel le bouton rotatif (400) définit en outre un canal central (404) à travers celui-ci, lequel canal central est conçu pour recevoir de manière sélective un entraînement de fixation (500) à travers celui-ci pour serrer et/ou desserrer les fixations supérieure et inférieure (224c, 225c) respectives qui s'accouplent aux cabestans (224b, 224b) pour faciliter davantage les réglages de tension.

3. Ensemble adaptateur (200) selon la revendication 1 ou 2, comprenant en outre un ensemble de mise à feu (230) qui s'étend à travers l'ensemble d'entraînement à câble (220) et dans le tube extérieur (204).

4. Ensemble adaptateur (200) selon la revendication 3, dans lequel l'ensemble de mise à feu (230) comprend un arbre de mise à feu (232) qui tourne indépendamment de l'ensemble d'entraînement à câble (220) pour actionner une fonction de mise à feu de l'effecteur d'extrémité (300).

5. Ensemble adaptateur (200) selon l'une quelconque des revendications précédentes, dans lequel le boîtier (202) comprend un boîtier extérieur (202b) et un boîtier intérieur (202a), les boîtiers intérieur et extérieur (202a, 202b) supportant l'ensemble d'entraînement à câble (220) à l'intérieur de ceux-ci.

6. Ensemble adaptateur (200) selon l'une quelconque des revendications précédentes, dans lequel l'ensemble d'entraînement à câble (220) comprend en outre au moins une poulie (228a-d, 229a-d) supportant les câbles (240a, 240b) et conçue pour diriger les câbles (240a, 240b) dans le tube extérieur (204).

7. Ensemble adaptateur (200) selon l'une quelconque des revendications précédentes, dans lequel l'ensemble d'entraînement à câble (220) comprend en outre une partie de corps (222) qui supporte les engrenages à vis sans fin (226d, 227d) et les engrenages à câble (224a, 225a) en relation de contact les uns avec les autres.

8. Ensemble adaptateur (200) selon l'une quelconque des revendications précédentes, dans lequel le tube extérieur (204) définit un axe longitudinal qui s'étend entre les extrémités proximale et distale (206) du tube extérieur, et dans lequel les engrenages à vis sans fin (226d, 227d) sont supportés par des éléments d'arbre (226e, 227e) qui s'étendent parallèlement à l'axe longitudinal du tube extérieur (204), les éléments d'arbre (226e, 227e) pouvant tourner pour faire tourner les engrenages à vis sans fin (226d, 227d).

9. Appareil d'agrafage chirurgical, comprenant :
un effecteur d'extrémité (300) ;
un dispositif chirurgical conçu pour actionner l'effecteur d'extrémité (300) ; et
un ensemble adaptateur (200) selon l'une quelconque des revendications précédentes.
